# EUROPÄISCHE PATENTSCHRIFT

(11) **EP 1 224 385 B1**
(45) Veröffentlichungstag und Bekanntmachung des Hinweises auf die Patenterteilung: **01.12.2004**
(21) Anmeldenummer: 00967691.7
(22) Anmeldetag: 18.09.2000
(51) Int. Cl.: F01N 9/00

(54) **VERFAHREN ZUR DIAGNOSE EINES SCHÄDIGUNGSZUSTANDES EINES IN EINEM ABGASKANAL EINER VERBRENNUNGSKRAFTMASCHINE ANGEORDNETEN NOx-SPEICHERKATALYSATORS**
METHOD FOR DIAGNOSING THE LEVEL OF DETERIORATION OF AN NOx CATALYST LOCATED IN THE EXHAUST CHANNEL OF AN INTERNAL COMBUSTION ENGINE
PROCEDE POUR ETABLIR UN DIAGNOSTIC SUR UN ETAT DE DETERIORATION DANS UN POT CATALYTIQUE A ACCUMULATION DE NOx MONTE DANS UN CANAL DE GAZ D'ECHAPPEMENT D'UN MOTEUR A COMBUSTION INTERNE

(30) Priorität: 29.09.1999 DE 19946628
(43) Veröffentlichungstag der Anmeldung: 24.07.2002
(73) Patentinhaber: Volkswagen Aktiengesellschaft, 38436 Wolfsburg (DE)
(72) Erfinder: LOECK, Harald, 38442 Wolfsburg (DE); KREBS, Rudolf, 38176 Wendeburg (DE); KÖNIG, Axel, 38448 Wolfsburg (DE); DRÜCKHAMMER, Jens, 38108 Braunschweig (DE)
(74) Vertreter: Pohlmann, Bernd Michael
(86) Internationale Anmeldenummer: PCT/EP2000/009093
(87) Internationale Veröffentlichungsnummer: WO 2001/023875

(56) Entgegenhaltungen:
- EP-A- 0 690 213
- EP-A- 0 756 072
- EP-A- 0 916 941
- DE-A- 19 746 658
- DE-A- 19 751 895
- US-A- 5 743 084

## Beschreibung

Die Erfindung betrifft ein Verfahren zur Diagnose eines Schädigungszustandes eines in einem Abgaskanal einer Verbrennungskraftmaschine angeordneten NOₓ-Speicherkatalysators mit den im Oberbegriff des Anspruchs 1 genannten Merkmalen.

Zur Reduzierung einer Emission von während eines Verbrennungsvorganges eines Luft-Kraftstorf-Gemisches in der Verbrennungskraftmaschine gebildeter Schadstoffe ist es bekannt, Katalysatoren in dem Abgaskanal anzuordnen. Dabei werden Schadstoffe, die als Reduktionsmittel dienen können, wie CO, HC oder H₂, mit vorhandenem Luftsauerstoff oxidiert. Ebenfalls während des Verbrennungsvorganges gebildetes NOₓ wird an dem Katalysator mit Hilfe der Reduktionsmittel zu Stickstoff reduziert.

Befindet sich die Verbrennungskraftmaschine in einem Magerbetrieb mit λ > 1, so ist ein Anteil der Reduktionsmittel am Abgas ebenfalls vermindert und eine vollständige Umsetzung des NOₓ kann nicht mehr erfolgen. Zur Abhilfe ist dem Katalysator eine Speicherkomponente für NOₓ zugeordnet, wobei beide als NOₓ-Speicherkatatysator zusammengefaßt werden können. Ein solcher NOₓ-Speicherkatalysator kann auch Bestandteil eines 3-Wege-Katalysators sein. Eine Absorption des NOₓ findet so lange statt, bis entweder eine Katalysatortemperatur eine NOₓ-Desorptionstemperatur überschreitet oder eine NOₓ-Speicherfähigkeit erschöpft ist. Vor diesem Zeitpunkt muß der NOₓ-Speicherkatalysator regeneriert werden, indem ein Wechsel in einen stöchiometrischen Betrieb (λ = 1) oder Fettbetrieb (λ < 1) eingeleitet wird.

Weiterhin ist bekannt, der Verbrennungskraftmaschine Mittel zuzuordnen, die es erlauben, insbesondere nach einem Kaltstart der Verbrennungskraftmaschine, den NOₓ-beispielsweise durch eine Anordnung einer Sekundärluftpumpe, die einen Sauerstoffanteil im Abgas durch kontrollierte Einspeisung eines Luftvolumens erhöht, und eine darauf abgestimmte Einstellung des motorischen Luftverhältnisses, erfolgen. Nachfolgend wird auf diese Weise eine Abgastemperaturerhöhung infolge exothermer Reaktionen erreicht.

Ferner ist bekannt, stromab des NOₓ-Speicherkatalysators einen NOₓ-Sensor anzuordnen, der eine NOₓ-Konzentration im Abgas erfaßt. Über die NOₓ-Konzentration kann dann in bekannter Weise eine Regenerationsnotwendigkeit, beispielsweise nach dem Überschreiten einer vorgebbaren Schwellenemission, eingeleitet werden.

Während eines Betriebes des NOₓ-Speicherkatalysators kann eine Funktionalität des NOₓ-Speicherkatalysators durch verschiedene Schädigungen beeinträchtigt werden. Zum einen können reversible Schädigungen, wie eine Verschwefelung durch schwefelhaltige Kraftstoffe oder eine Belegung des NOₓ-Speicherkatalysators durch Ruß, mit Hilfe gezielter Regenerationsmaßnahmen wieder behoben werden. Zum anderen treten mit zunehmender Betriebsdauer aber auch irreversible Schädigungen in Erscheinung. So kann beispielsweise durch thermische Überbelastung ein Aktivitätsverlust des NOₓ- Speicherkatalysators eintreten und damit gegebenenfalls eine wirksame Reduktion der Schadstoffemissionen nicht mehr ermöglicht werden.

Femer ist aus der DE 197551895 A1 ein Katalysator-Diagnoseverfahren bekannt, bei dem der Istwert eines Signals einer stromabwärts des Katalysators angeordneten Lambdasonde erfasst wird und bei dem ein Erwartungswert des Signals der genannten Abgassonde und ein Maß für die Abweichung des Istwertes vor dem Erwartungswert gebildet wird. Die Funktionsfähigkeit des Katalysators wird auf der Basis diese Maßes beurteilt, wobei von der hinter dem Katalysator angeordneten Lambdasonde Oszillationen des Sauerstoffgehaltes des Abgases erfasst werden. Unerwartet starke Oszillationen des Sauerstoffgehaltes deuten auf eine reduzierte Sauerstoff-Speicherfähigkeit des Katalysators hin und werden als Zeichen für einen defekten Katalysator gewertet.

Nachteilig bei den bekannten Verfahren zur Steuerung von NOₓ-Speicherkatalysatoren ist es, daß derartige Schädigungszustände nur unzureichend diagnostiziert werden, weil sie nur bei betriebswarmem Motor stattfinden. Findet eine Diagnose statt, so wird häufig auf relativ komplexe Meßanordnungen, die zusätzliche Lambdasensoren, Temperatursensoren und spezifische Gassensoren umfassen, zurückgegriffen. Dies erfordert zum einen ein komplexes Auswerteverfahren, und zum anderen sind derartige Sensoren relativ kostspielig und erhöhen somit die Fertigungskosten erheblich.

Der Erfindung liegt die Aufgabe zugrunde, ein Verfahren zur Verfügung zu stellen, das die Diagnose des Schädigungszustandes des NOₓ-Speicherkatalysators in einfacher Weise und unter Zuhilfenahme von nur einem NOₓ-Sensor ermöglicht.

Erfindungsgemäß wird diese Aufgabe durch das Verfahren zur Diagnose des Schädigungszustandes des NOₓ-Speicherkatalysators mit den im Anspruch 1 genannten Merkmalen gelöst. Dadurch, daß
(a) nach Beendigung der Aufheizmaßnahme (aus motorseitigen Maßnahmen und der Sekundärlufteinblasung) innerhalb eines vorgebbaren Diagnosezeitraumes ein Signalverlauf der NOₓ-Konzentration von dem NOₓ-Sensor erfaßt wird und
(b) der Signalverlauf der NOₓ-Konzentration mit einem vorgebbaren Sollverlauf für die NOₓ-Konzentration verglichen wird,
kann bereits unmittelbar nach dem Kaltstart der Verbrennungskraftmaschine und dem Aufheizen des Speicherkatalysators auf die Mindesttemperatur der Schädigungszustand erfaßt werden.

In bevorzugter Weise wird ein Verlauf und/oder eine Größe einer Abweichung des Signalverlaufes von dem Sollverlauf zu einem Kontrollwert zusammengefaßt Überschreitet dabei der Kontrollwert einen vorgebbaren Schwellenwert, so kann gegebenenfalls ein Wartungssignal erzeugt werden (On-Board-Diagnose). Anhand des Wartungssignales können dann zum einen entsprechende Regenerationsmaßnahmen ergriffen werden oder zum anderen umfangreichere Wartungsmaßnahmen eingeleitet werden. Dabei hat es sich als vorteilhaft erwiesen, den Schwellenwert anhand von Parametern, wie eine Gesamtbetriebsdauer des NOₓ-Speicherkatalysators, eine Effizienz vorhergehender Regenerationsmaßnahmen, eine Fahrzeuggeschwindigkeit oder einer aktuellen Katalysatortemperatur, festzulegen.

Als besonders günstig hat es sich erwiesen, das erfindungsgemäße Verfahren an Verbrennungskraftmaschinen durchzuführen, denen eine Sekundärluftpumpe zugeordnet ist. Die Sekundärluftpumpe dient in bekannter Weise nach dem Kaltstart der Verbrennungskraftmaschine zur Einleitung der Aufheizmaßnahme, bei der die Katalysatortemperatur auf die Mindestbetriebstemperatur erhöht wird. Nach der Abschaltung der Sekundärluftpumpe wird dann die Diagnose eingeleitet.

Eine Diagnose ist erst nach dem Abschalten der Sekundärluftpumpe sinnvoll, weil ab diesem Zeitpunkt die Gemischregeliung auf λ = 1 eingestellt werden kann. Unter dem λ = 1 geregelten Betriebsmodus setzt dann die NOₓ-Reduktion des Katalysators ein. Unter diesen Betriebsbedingungen kann eine eventuell vorhandene Schädigung des Katalysators mit Hilfe des NOₓ-Sensors erfaßt werden.
Die Diagnose des Schädigungszustandes wird femer vorzugsweise innerhalb eines vorgebbaren Temperaturbereiches für die Katalysatortemperatur durchgeführt, wobei die Katalysatortemperatur durch geeignete Temperatursensoren erfaßt werden kann.

Weitere bevorzugte Ausgestaltungen der Erfindung ergeben sich aus den übrigen, in den Unteransprüchen genannten Merkmalen.

Die Erfindung wird nachfolgend in einem Ausführungsbeispiel anhand der zugehörigen Zeichnung, die einen Verlauf eines Lambdawertes, einen Verlauf einer Fahrgeschwindigkeit, einen Signalverlauf einer NOₓ-Konzentration und einen vorgebbaren Sollverlauf für die NOₓ-Konzentration zeigt, näher erläutert.

Eine Verbrennungskraftmaschine, insbesondere ein λ = 1 geregelter oder DI-Otto-Motor, emittiert während eines Betriebes Schadstoffe, wie CO, HC und NOₓ. Einerseits können über in einem Abgaskanal der Verbrennungskraftmaschine angeordnete Katalysatoren Reduktionsmittel (CO, HC, H₂) mit Sauerstoff oxidiert werden, und andererseits kann mit Hilfe der Reduktionsmittel NOₓ an den Katalysatoren zu Stickstoff umgesetzt werden. In einem Magerbetrieb mit λ > 1 wird NOₓ als Nitrat in einem NOₓ-Speicherkatalysator eingelagert, und zwar solange, bis entweder eine NOₓ-Desorptionstemperatur überschritten wird oder eine NOₓ-Speicherfähigkeit erschöpft ist. Zur Vermeidung eines NOₓ-Durchbruches wird die Verbrennungskraftmaschine vor diesem Zeitpunkt unter stöchiometrischen oder fetten Bedingungen mit λ ≤ 1 (Regeneration) betrieben,

Nach einem Kaltstart der Verbrennungskraftmaschine wird üblicherweise ein Betriebsmodus der Verbrennungskraftmaschine mit λ < 1 (Fettbetrieb) eingestellt (siehe Verlauf des Lambdawertes 10), um ein schnelleres Aufheizen des NOₓ-Speicherkatalysators zu erreichen. Zusätzlich wird gegebenenfalls während einer Beschleunigung eines durch die Verbrennungskraftmaschine angetriebenen Kraftfahrzeuges (siehe Verlauf der Fahrzeuggeschwindigkeit 12) der Fettbetrieb erzwungen.

Der NOₓ-Speicherkatalysator muß zur Gewährung einer hinreichenden Konvertierungsrate für NOₓ Und NOₓ-Speicherfähigkeit eine Mindestbetriebstemperatur aufweisen. Dazu sind der Verbrennungskraftmaschine Mittel zugeordnet, die insbesondere nach einem Kaltstart eine Einstellung der Katalysatortemperatur durch eine zumindest temporäre Beeinflussung der Abgastemperatur erlauben (Aufheizmaßnahme während einer Phase t_{S}). So kann durch eine Sekundärluftpumpe ein Sauerstoffanteil am Abgas durch Einspeisung eines zusätzlichen Luftvolumens unmittelbar nach der Verbrennungskraftmaschine erhöht werden. Befindet sich die Verbrennungskraftmaschine im Fettbetrieb, erfolgt somit eine verlagerte exotherme Oxidation der Reduktionsmittel direkt im oberen Abgaskanal oder gegebenenfalls an einem vorhanden Vorkatalysator.

Nach Beendigung der Aufheizmaßnahme, beispielsweise nachdem eine Katalysatortemperatur in einem vorgebbaren Temperaturbereich liegt, wird innerhalb eines vorgebbaren Diagnosezeitraumes (Phase t_{D}) über einen stromab des NOₓ-Speicherkatalysators angeordneten NOₓ-Sensor der Signalverlauf 16 der NOₓ-Konzentration erfaßt. Ein derartiger Signalverlauf 16 kann beispielsweise in ein Motorsteuergerät eingelesen werden und wird mit einem dort hinterlegten vorgebbaren Sollverlauf 14 für die NOₓ-Konzentration verglichen. Der Sollverlauf 14 zeigt dabei üblicherweise den Verlauf der NOₓ-Konzentration eines frischen NOₓ-Speicherkatalysators.

Ein Verlauf und/oder eine Größe einer Abweichung des Signalverlaufs 16 von dem Sollverlauf 14 liefert somit ein Maß für den Schädigungszustand des NOₓ-Speicherkatalysator. Der Verlauf und/oder die Größe der Abweichung können zu einem Kontrollwert zusammengefaßt werden. Beim Überschreiten des Kontrollwertes über einen Schwellenwert kann dann ein Wartungssignal erzeugt werden. Dabei kann der Schwellenwert mit Hilfe von Parametem, wie einer Gesamtbetriebsdauer des Katalysators, einer Effizienz vorhergehender Regenerationsmaßnahmen der Fahrzeuggeschwindigkeit 12 oder der Katalysatortemperatur, festgelegt werden.

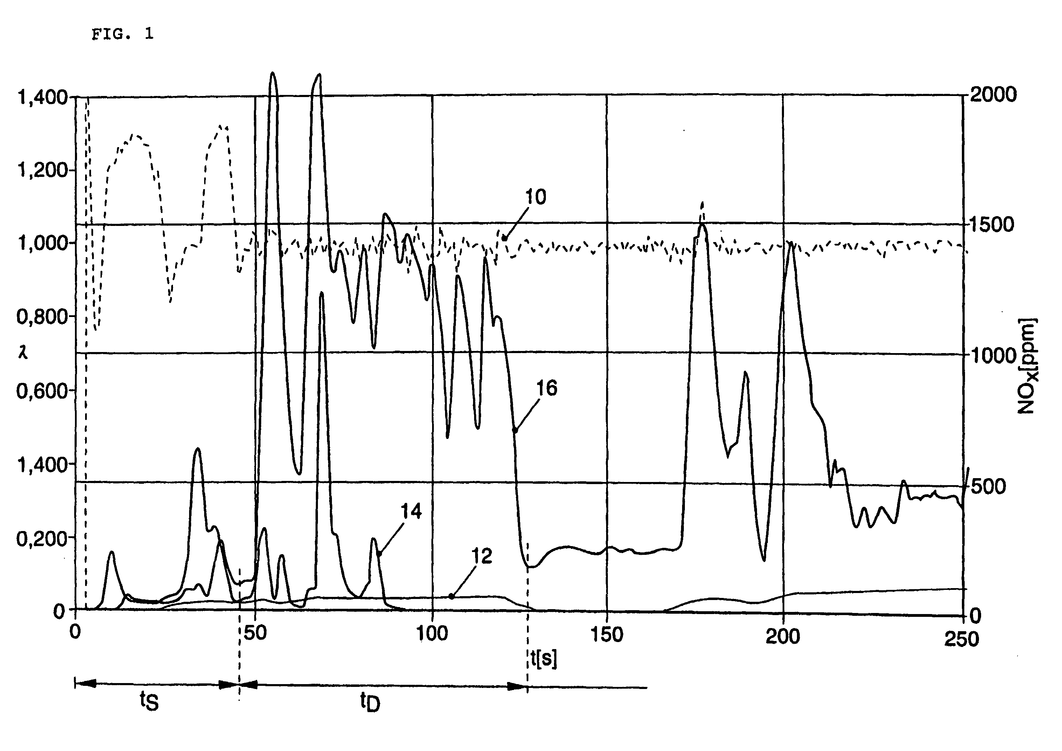

## Patentansprüche

1. Verfahren zur Diagnose eines Schädigungzustandes eines in einem Abgaskanal einer Verbrennungskraftmaschine angeordneten NOₓ-Speicherkatalysators mit einem stromab des des NOₓ-Speicherkatalysators angeordneten NOx-Sensors zur Erfassung einer NOₓ-Konzentration und der Verbrennungskraftmaschine zugeordneten Mitteln zur Einstellung der Katalysatortemperatur durch eine zumindest temporäre Beeinflussung der Abgastemperatur, bei dem
- durch eine Aufheizmaßnahme die Katalysatortemperatur auf einen Wert innerhalb eines vorgebbaren Temperaturbereichs eingestellt wird,
- nach Beendigung der Aufheizmaßnahme innerhalb eines vorgegebenen Diagnosezeitraums ein der erfassten NOx-Konzentration entsprechender Signalverlauf (16) des NOx-Sensors ermittelt, mit einem vorgebbaren Sollverlauf (14) für die NOx-Konzentration verglichen und aus dem Vergleichsergebnis ein Maß für den Schädigungszustand des NOx-Speicherkatalysators gewonnen wird.

2. Verfahren nach Anspruch 1, **dadurch gekennzeichnet, daß** nach Beendigung der Aufheizmaßnahme die Verbrennungskraftmaschine in einem Betriebsmodus mit einem Lambdawert gleich 1 betrieben wird.

3. Verfahren nach Anspruch 1 oder 2, **dadurch gekennzeichnet, daß** nach einem Kaltstart der Verbrennungsmaschine und **/** oder ggf. während einer Beschleunigung eines durch die Verbrennungskraftmaschine angetriebenen Kraftfahrzeuges die Verbrennungskraftmaschine mit einem Betriebsmodus mit einem Lambdawert kleiner 1 betrieben wird.

4. Verfahren nach einem der vorhergehenden Ansprüche, **dadurch gekennzeichnet, daß** ein Verlauf und/oder eine Größe einer Abweichung des Signalverlaufes (16) von dem Sollverlauf (14) zu einem Kontrollwert zusammengefaßt wird.

5. Verfahren nach Anspruch 4, **dadurch gekennzeichnet, daß** beim Überschreiten des Kontrollwertes über einen Schwellenwert ein Wartungssignal erzeugt wird.

6. Verfahren nach Anspruch 5, **dadurch gekennzeichnet, daß** der Schwellenwert anhand von Parametern, wie einer Gesamtbetriebsdauer des NOₓ-Speicherkatalysa-tors, einer Effizienz vorhergehender Regenerationsmaßnahmen, einer Fahrzeuggeschwindigkeit oder der aktuellen Katalysatortemperatur, festgelegt wird

7. Verfahren nach einem der vorhergehenden Ansprüche, **dadurch gekennzeichnet,** das die Mittel zur Einstellung der Mindestbetriebstemperatur des NOₓ-Speicherkatalysators eine Sekundärluftpumpe umfassen.

8. Verfahren nach einem der vorhergehenden Ansprüche, **dadurch gekennzeichnet, daß** die Diagnose des Schädigungszustands innerhalb eines vorgebbaren Temperaturbereiches für eine Katalysatortemperatur erfolgt.

## Claims

1. Method for diagnosing a damage state of an NOx storage catalytic converter arranged in an exhaust-gas duct of an internal combustion engine, with an NOx sensor arranged downstream of the NOx storage catalytic converter for recording an NOx concentration and with means assigned to the internal combustion engine for setting the catalytic converter temperature by at least temporarily influencing the exhaust-gas temperature, in which method
- the catalytic converter temperature is set, by a heating measure, to a value which lies within a predeterminable temperature range,
- after the heating measure has ended, a signal curve (16) from the NOx sensor which corresponds to the NOx concentration recorded is determined within a predetermined diagnosis period, compared with a predeterminable desired curve (14) for the NOx concentration, and a measure of the damage state of the NOx storage catalytic converter is obtained from the result of the comparison.

2. Method according to Claim 1, **characterized in that** after the heating measure has ended, the internal combustion engine is operated in an operating mode with a lambda value equal to 1.

3. Method according to Claim 1 or 2, **characterized in that** after a cold start of the internal combustion engine and/or if appropriate during acceleration of a motor vehicle driven by the internal combustion engine, the internal combustion engine is operated with an operating mode with a lambda value of less than 1.

4. Method according to one of the preceding claims, **characterized in that** a curve and/or a variable for a deviation in the signal curve (16) from the desired curve (14) is combined to form a monitoring value.

5. Method according to Claim 4, **characterized in that** a maintenance signal is generated if the monitoring value exceeds a threshold value.

6. Method according to Claim 5, **characterized in that** the threshold value is determined on the basis of parameters such as a total operating time of the NOx storage catalytic converter, an efficiency of previous regeneration measures, a vehicle speed or the current catalytic converter temperature.

7. Method according to one of the preceding claims, **characterized in that** the means for setting the minimum operating temperature of the NOx storage catalytic converter comprise a secondary air pump.

8. Method according to one of the preceding claims, **characterized in that** the diagnosis of the damage state takes place within a predeterminable temperature range for a catalytic converter temperature.

## Revendications

1. Procédé de diagnostic d'un état de détérioration d'un catalyseur d'accumulation de NOₓ disposé dans la conduite d'échappement d'un moteur à combustion interne, avec un capteur de NOₓ disposé en aval du catalyseur d'accumulation de NOₓ pour détecter une concentration en NOₓ et avec des moyens associés au moteur à combustion interne pour le réglage de la température du catalyseur par une influence au moins temporaire de la température des gaz d'échappement, dans lequel
- la température du catalyseur est réglée à une valeur située dans une plage de températures prédéfinissable, par une mesure d'échauffement,
- à l'issue de la mesure d'échauffement, au cours d'une période de diagnostic prédéfinie, une courbe de signaux (16) du capteur de NOₓ correspondant à la concentration en NOₓ détectée est déterminée, comparée à une courbe théorique prédéfinissable (14) pour la concentration en NOₓ et une dimension pour l'état de détérioration du catalyseur d'accumulation de NOₓ est déterminée à partir du résultat de la comparaison.

2. Procédé selon la revendication 1, **caractérisé en ce qu'**après achèvement de la mesure d'échauffement, le moteur à combustion interne est exploité à un régime d'exploitation avec une valeur lambda égale à 1.

3. Procédé selon la revendication 1 ou 2, **caractérisé en ce qu'**après un démarrage à froid du moteur à combustion interne et/ou le cas échéant au cours d'une accélération d'un véhicule automobile entraîné par le moteur à combustion interne, le moteur à combustion interne est exploité à un régime d'exploitation avec une valeur lambda inférieure à 1.

4. Procédé selon l'une quelconque des revendications précédentes, **caractérisé en ce qu'**une courbe et/ou une dimension d'un écart de la courbe de signaux (16) par rapport à la courbe théorique (14) est regroupée en une valeur de contrôle.

5. Procédé selon la revendication 4, **caractérisé en ce que** lors du dépassement de la valeur de contrôle au-dessus d'une valeur seuil, un signal de maintenance est délivré.

6. Procédé selon la revendication 5, **caractérisé en ce que** la valeur seuil est déterminée à l'aide de paramètres, comme une durée d'exploitation totale du catalyseur d'accumulation de NOₓ, une efficacité de mesures de régénération antérieures, une vitesse de conduite ou la température actuelle du catalyseur.

7. Procédé selon l'une quelconque des revendications précédentes, **caractérisé en ce que** les moyens de réglage de la température de régime minimale du catalyseur d'accumulation de NOₓ comprennent une pompe à air secondaire.

8. Procédé selon l'une quelconque des revendications précédentes, **caractérisé en ce que** le diagnostic de l'état de détérioration a lieu dans une plage de températures prédéfinissable pour une température du catalyseur.
